# EUROPÄISCHE PATENTANMELDUNG

(11) **EP 3 915 801 A1**
(43) Veröffentlichungstag der Anmeldung: **01.12.2021**
(21) Anmeldenummer: 21175894.1
(22) Anmeldetag: 26.05.2021
(51) Int. Cl.: B43K 23/00, A61L 2/10

(54) **AUFNAHMEHÜLSE**

(30) Priorität: 28.05.2020 DE 202020103065 U; 15.10.2020 DE 202020105889 U
(71) Anmelder: Hillemann Design Markus Hillemann & Ute Walliser GbR, 85354 Freising (DE)
(72) Erfinder: HILLEMANN, Markus, 85402 Kranzberg (DE)
(74) Vertreter: Tergau & Walkenhorst Patentanwälte PartGmbB

(57) **Zusammenfassung**

Aufnahmehülse für einen Stift (4) mit einer den Stift (4) über seine gesamte Länge aufnehmenden Aufnahmekammer (3) mit einer reflektierenden Innenwand (5) und mit einer oder mehreren Strahlungsquellen (6) an der Innenwand (5), mit einer verschließbaren Einführöffnung (17) am freien Aufnahmekammerende (3), mit einem Auswerfer an dem der Einführöffnung (17) abgewandten festen Ende der Aufnahmekammer (3) und mit mindestens einem Zentrierelement zur Zentrierung des Stiftes (4) entlang der Mittellängsachse (13) der Aufnahmekammer (3).

## Beschreibung

Die vorliegende Erfindung betrifft eine Aufnahme für einen Stift, nämlich eine Aufnahmehülse, welche geeignet ist, einen in ihr einliegenden Stift zu desinfizieren. Im Bereich des Handels und der Dienstleistungen aber auch im industriellen Bereich besteht die Notwendigkeit, Stifte vorzuhalten, um entweder Checklisten zu bearbeiten und/ oder zu signieren, Unterschriften zu leisten oder um mit Eingabestiften auf Touchpanels zu unterschreiben oder persönliche Identifikationsnummern in Touchpanels einzugeben, insbesondere einzutippen. Die Erfindung wendet sich also insbesondere an den Einzelhandel mit Kassentresen bzw. SB-Kassen, an Banken, Behörden, Kurierdienste und vieles andere mehr.

Dabei wird ein Stift in der Regel von vielen Personen nacheinander in die Hand genommen und berührt. Hierbei ist es unvermeidlich, dass sich an den Händen befindliche Keime, Bakterien, Verunreinigungen oder ähnliches auf der Außenhaut des Stiftes ablagern. Geht ein solcher Stift von Hand zu Hand der verschiedenen Benutzer, ist das daraus resultierende Risiko einer Berührungsinfektion evident.

Aus der US 9,566,819 B2 ist eine Reinigungsvorrichtung für mehrere Stifte bekannt. Die Stifte werden dort in einem nach Art einer senkrecht stehenden Revolvertrommel ausgebildeten Drehmagazin gelagert und in unterschiedlichen Drehstellungen gereinigt und desinfiziert. Um die Stifte in ihrer jeweiligen Aufnahmekammer zu zentrieren sind dort radial in die Aufnahmekammer hineinstehende Zentrierdorne vorgesehen.

Sowohl diese Zentrierdorne als auch der übrige Aufbau der Aufnahmekammern bzw. des revolvertrommelartigen Aufnahmemagazins haben den Nachteil, dass die zur Desinfektion der Stifte vorgesehenen Ultraviolettstrahler nicht sämtliche Bereiche der Mantelfläche des zu desinfizierenden Stifts erreichen. Es treten dort an einigen Stellen Verschattungen auf, die letztlich dazu führen, da einzelne Bereiche der Stifte nur unzureichend oder gar nicht bestrahlt und damit desinfiziert werden.

Ausgehend hiervon liegt der Erfindung die Aufgabe zugrunde, eine Aufnahmehülse für einen Stift so zu gestalten, dass sämtliche Bereiche der Mantelfläche des Stiftes homogen und gleichmäßig mit den Strahlen aus der Strahlungsquelle, insbesondere UV-Strahlungsquelle beaufschlagt werden.

Diese Aufgabe ist durch die Merkmalskombination des Anspruchs 1 in erfinderischer Weise gelöst. Die auf den Anspruch 1 zurückbezogenen Ansprüche beinhalten teilweise vorteilhafte und teilweise für sich selbst erfinderische Weiterbildungen der dort beanspruchten Erfindungen.

Die Erfindung geht davon aus, einen zu desinfizierenden Stift in der Aufnahmekammer einer Aufnahmehülse zu zentrieren, ohne dass hierdurch Teilbereiche der Mantelfläche des Stifts bedeckt oder verdeckt werden. Zur Realisierung dieser Überlegung wird der zu desinfizierende Stift mit einem seiner Enden oder mit seinen beiden Enden in der Aufnahmekammer der Aufnahmehülse zentriert. Um zu verhindern, dass UV-Licht als Streulicht die als Desinfektionskammer wirksame Aufnahmekammer der Aufnahmehülse verlässt, ist die Einführöffnung am freien Ende der Aufnahmekammer verschließbar. Der Verschluss der Einführöffnung verhindert zum einen das Austreten von Strahlung. Zum anderen ist der Verschluss in einer bevorzugten Ausführungsform an seinen Innenflächen reflektierend ausgebildet, sodass auf die Verschlussinnenwände auftreffende Strahlen wieder in Richtung auf den desinfizierenden Stift reflektiert werden.

Um den Stift nach der Desinfektion aus der Aufnahmehülse wieder entnehmen zu können, ist weiterhin ein Auswerfer vorgesehen. Dieser Auswerfer ist an dem dem freien Aufnahmekammerende mit der Einführöffnung abgewandten festen Ende der Aufnahmekammer ausgebildet. Der Auswerfer beaufschlagt die Stiftspitze des desinfizierten Stiftes und verfährt den Stift entlang seiner Mittellängsachse und damit entlang der Mittellängsachse der Aufnahmekammer nach oben in Richtung auf das freie Aufnahmekammerende. Vor dem Verfahren muss der Verschluss an der Einführöffnung geöffnet werden, sodass ein Teilbereich des Stiftes aus der Aufnahmekammer der Aufnahmehülse herausfährt und von der Hand des Benutzers leicht gegriffen werden kann.

Schließlich ist noch mindestens ein Zentrierelement in der Aufnahmekammer der Aufnahmehülse angeordnet. Dieses Zentrierelement wirkt entweder auf die Stiftspitze oder auf das der Stiftspitze abgewandte Stiftende ein. Es können auch mehrere Zentrierelemente vorgesehen sein, die sowohl dass Stiftende als auch die Stiftspitze beaufschlagen.

In vorteilhafte Ausgestaltung ist eine trichterförmige Aufnahmefläche für die Stiftspitze am Auswerfer vorgesehen. Diese trichterförmige Aufnahmefläche zentriert den zu desinfizierenden Stift aufgrund der Trichterform und der Schwerkraft selbsttätig in einer Stellung, in der die Mittellängsachse des zu desinfizierenden Stiftes mit der Mittellängsachse der Aufnahmekammer deckungsgleich ist. Weist der zu desinfizierende Stift an seiner Stiftspitze eine hinausstehende Mine, bspw. den Kopf einer Kugelschreibermine auf, ist im Zentrum der trichterförmigen Aufnahmefläche eine zentrale Freimachung vorgesehen, in welche die Stiftspitze bzw. Minenspitze einfach eintauchen kann. Die Minenspitze bildet mit der Freimachung eine Art Nut-Feder-Verbindung aus, sodass die Stiftspitze dann formschlüssig an der trichterförmigen Aufnahmefläche gelagert ist.

Eine erste Variante eines Zentrierelements ist eine an dem der Stiftspitze abgewandten Schaftende des Stiftes adaptierte Halteschnur oder Haltekordel. Die Halteschnur oder Haltekordel durchsetzt den geschlossenen Verschluss der Aufnahmekammer durch eine Zentrieröffnung hindurch, die im Verschluss zentral freigelassen ist. Der Verschluss mit seiner Zentrieröffnung hat im Zusammenwirken mit der Halteschnur oder Haltekordel somit die Doppelfunktion eines Verschlusses einerseits und eines Zentrierelements andererseits.

Eine weitere Variante des Zentrierelements ist ein an dem der Stiftspitze abgewandten Schaftende des Stiftes ausgebildeter Zentriervorsprung, welcher in eine zentrale, im Verschluss freigelassene Zentrieröffnung eingreift. Das Schaftende des Stiftes ist somit im Verschluss formschlüssig gelagert. Der Verschluss mit seiner Zentrieröffnung hat somit die Doppelfunktion eines erfindungsmäßigen Verschlusses einerseits und eines erfindungsmäßigen Zentrierelements andererseits.

Besonders vorteilhaft ist eine Zwei-Punkt-Zentrierung mit der vorstehend beschriebenen trichterförmigen Aufnahmefläche ggfs. mit Freimachung einerseits und dem ebenfalls vorbeschriebenen Verschluss mit Doppelfunktion, nämlich einer zentralen, einen am Stift ausgebildeten Zentriervorsprung umfassenden Zentrieröffnung andererseits.

Der Vorteil dieser Zentrierung besteht darin, dass die gesamte Außenfläche bzw. Mantelfläche des zu desinfizierenden Stifts vollständig von den UV-Strahlungsquellen beleuchtet und damit mit UV-Strahlung beaufschlagt wird. Insbesondere ist die Bildung von Schatten auf diese Weise wirksam verhindert. In diesem Zusammenhang ist es vorteilhaft, sowohl die trichterförmige Aufnahmefläche als auch die Innenseiten des Verschlusses als auch die Innenwände der Aufnahmekammer aus einem diffus reflektierenden Material zu fertigen bzw. mit einem solchen Material zu beschichten. Besonders gut geeignet als Oberfläche ist matt eloxiertes Aluminium. Sowohl die Innenseiten der Lamellen am Verschluss als auch die trichterförmig ausgestaltete Auflage leiten die auftreffende UV-Strahlung in Richtung auf die Außenfläche des zu desinfizierenden Stiftes und tragen damit zur vollständigen Desinfektion des Stiftes bei.

Die Freimachung in der Aufnahmefläche verhindert bei einem aus der Stiftspitze hinausstehenden Kopf einer Kugelschreibermine ein Verkleben des Schreibkopfes der Kugelschreibermine.

In einer bevorzugten Ausführungsvariante ist der am Stiftende ausgebildete Zentriervorsprung zugleich als Halteöse für eine Halteschnur, Haltekette oder dergleichen für den Stift ausgestaltet. Auf diese Weise ist ausgeschlossen, dass der Stift ungewollt von der Aufnahmehülse entfernt, insbesondere gestohlen wird. Außerdem ist so wirksam verhindert, dass ein ordnungsgemäß desinfizierter Stift gegen einen nicht desinfizierten Stift unbemerkt ausgetauscht wird. Auf diese Weise ist sichergestellt, dass jeder aus der Aufnahmehülse ausgeworfene Stift mit 100 prozentiger Sicherheit vollständig desinfiziert ist.

In weiterer bevorzugter Ausgestaltung wird der Verschluss am freien Ende der Aufnahmehülse von zwei oder mehr Lamellen gebildet. Diese Lamellen überlappen einander im verschlossenen Zustand des Verschlusses. Es können auch mehrere radial zu einander verstellbare Lamellen vorgesehen sein. Der so ausgestaltete Verschluss ist nach Art einer Kamerablende ausgebildet. Die Verschlusslamellen können sowohl motorisch, insbesondere elektromotorisch angetrieben sein als mechanisch mit einem Hebel geöffnet und verschlossen werden.

In einer alternativen Ausgestaltung ist der Verschluss am Freiende der Aufnahmekammer von einem oder mehreren Bürstenringen gebildet. Die Borsten des oder der Bürstenringe sind an einer kreisförmigen Öffnung am freien Aufnahmekammerende angeordnet. Die Borsten der oder des Bürstenrings stehen dabei radial vom Öffnungsrand in die Öffnung hinein. Zum Einführen des Stiftes wird der Stift einfach durch die Borsten hindurchgeschoben, wobei sich die Borsten elastisch verformen und nach dem Ein- bzw. Durchschieben des Stiftes wieder in ihre Ausgangsstellung zurückbewegt.

Besonders vorteilhaft ist es, mehrere Bürstenringe mit verschieden langen Borsten miteinander zu kombinieren. Auf diese Weise ist sichergestellt, dass beim Rückverformen der Borsten, also im geschlossenen Zustand des Verschlusses kein UV-Licht aus der Aufnahmekammer nach Außen abstrahlen kann.

Schließlich ist es in einer weiteren Ausgestaltung auch möglich, den Verschluss aus einer Vielzahl flexibler Lippen, insbesondere Kunststofflippen herzustellen. Ein Lippenverschluss hat ebenso wie ein Lamellenverschluss den Vorteil, dass die der Aufnahmekammer zugewandten Bereiche der Lippen oder Lamellen aus einem reflektierenden Material gefertigt oder mit einem reflektierenden Material beschichtet werden können. Bei der Verwendung von Bürstenringen ist diese Beschichtung schwieriger realisierbar.

Der Auswerfer ist in bevorzugter Ausführungsform als Hubzylinder bzw. Teleskopzylinder ausgestaltet. Die Schnittstelle zwischen dem freien Ende des Hubzylinders und der Minenspitze bildet dann die vorzugsweise trichterförmige Aufnahmefläche für die Stiftspitze. In einer weiteren bevorzugten Ausgestaltung ist dieser Hubzylinder oder Teleskopzylinder elektromotorisch angetrieben. Der Hubzylinder bzw. Hydraulikzylinder kann auch hydraulisch oder pneumatisch angetrieben werden. Nach dem Desinfizieren werden die Aufnahmefläche und damit der Stift entlang der Mittellängsachse der Aufnahmekammer aus der Aufnahmekammer und damit der Aufnahmehülse heraus verfahren, um den Stift sodann mit der Hand greifen zu können.

Nach dem Entnehmen des Stiftes verfährt der Hubzylinder wieder in seine Ausgangsstellung und zieht die Aufnahmefläche in die Desinfektionsposition der Aufnahmehülse zurück. Ein weiterer zu desinfizierender Stift kann eingeschoben und durch Einschalten der UV-Strahlungsquellen desinfiziert werden.

In einer bevorzugten Variante kann es auch vorgesehen sein, den Hubzylinder bzw. Teleskopzylinder nach der Entnahme des Stiftes in seiner expandierten Entnahmestellung für den Stift zu belassen und ihn erst mit dem Einschieben des nächsten zu desinfizierenden Stiftes in seine eingefahrene Desinfektionsposition zurück zu verfahren. In weiterer bevorzugter Ausgestaltung ist es möglich, an der Aufnahmefläche schwenkbare Greifklauen vorzusehen, welche die Stiftspitze von mehreren Seiten nach Art eines Spannfutters umgreifen und gemeinsam mit dem Hubzylinder oder Teleskopzylinder in die Desinfektionsstellung zurück verfahren. Vor dem Desinfizieren müssen die Greifklauen wieder vom Stift weggeschwenkt werden, um Schattenbildung o. ä. zu verhindern, also um sicher zu stellen, dass weiterhin die Gesamte Außenfläche bzw. Mantelfläche des Stiftes von der UV-Strahlung direkt erfasst wird.

In einer weiteren Ausführung kann der Auswerfer anstelle des vorbeschriebenen Hub- oder Teleskopzylinders mit Antriebsspindeln auf und ab bewegt werden.

Anhand des nachfolgend beschriebenen Ausführungsbeispiels ist die Erfindung mit weiteren Einzelheiten erläutert. Es zeigen:
- Fig. 1: eine geschnittene Ansicht der Aufnahmehülse mit einem in der Aufnahmekammer der Aufnahmehülse einliegenden, zu desinfizierenden Stift,
- Fig. 2: eine perspektivisch geschnittene Ansicht der Aufnahmehülse mit einliegendem Stift in Desinfektionsstellung,
- Fig. 3: die Aufnahmehülse aus Fig. 2 mit ausgeworfenem Stift nach dem Desinfizieren,
- Fig. 4: die Aufnahmehülse nach dem Entnehmen des Stifts in expandierter Stellung des Hubzylinders und mit geöffnetem Verschluss an der Einführöffnung,
- Fig. 5: die Aufnahmehülse aus Fig. 4 mit in die Desinfektionsstellung zurückgezogenem Hubzylinder und mit geschlossenem Verschluss an der Einführöffnung,
- Fig. 6: eine Detaildarstellung der trichterförmigen Aufnahmefläche am Auswerfer,
- Fig. 7: die Detaildarstellung aus Fig. 6 mit einliegender Stiftspitze,
- Fig. 8: eine Detaildarstellung des geöffneten Verschlusses an der Einführöffnung,
- Fig. 9: die Detaildarstellung aus Fig. 8 mit eingeführtem Stift, geschlossenem Verschluss und mit in der Zentrieröffnung des Verschlusses einliegendem Zentriervorsprung am Stift,
- Fig. 10: die Detaildarstellung eines aus drei Bürstenringen unterschiedlicher Größe gebildeten Verschlusses an der Einführöffnung,
- Fig. 11: eine schematische Darstellung der Aufnahmehülse mit einem von einer Antriebsspindel bewegten Auswerfer in seiner Desinfektionsstellung sowie
- Fig. 12: eine schematische Darstellung der Aufnahmehülse mit einem von einer Antriebsspindel bewegten Auswerfer in seiner Entnahmestellung.

Die Aufnahmehülse gemäß dem Ausführungsbeispiel besteht aus einem Standfuß 1 und einem zylinderförmigen Hülsenkörper 2. Der Hülsenkörper 2 umkleidet die hohlzylinderische Aufnahmekammer 3 für den zu desinfizierenden Stift 4. An der umlaufenden und aus gebürstetem Aluminium bestehenden Innenwand 5 der Aufnahmekammer 3 sind als UVC-Lichter ausgebildete Strahlungsquellen 6 angeordnet. Die Strahlungsquellen 6 sind über die gesamte Innenwand 5 der Aufnahmekammer 3 verteilt.

Der Stift 4 weist eine Stiftspitze 7 auf. Aus der Stiftspitze 7 steht der Minenkopf 8 einer Kugelschreibermine hinaus. Der Minenkopf 8 ist in eine Freimachung 9 an einem Aufnahmeblock 10 eingesteckt. Der Aufnahmeblock 10 weist seinerseits eine trichterförmig ausgebildete Aufnahmefläche 11 auf. Die trichterförmige Aufnahmefläche 11 bewirkt, dass die Stiftsspitze zentriert auf dem Aufnahmeblock 10 aufliegt und das der Minenkopf 8 direkt und widerstandslos in die Freimachung 9 hinein gleitet und in der Freimachung 9 formschlüssig einliegt. Die trichterförmige Aufnahmefläche 11 kann dabei in ihrer Form so an die Stiftspitze 7 angepasst sein, dass sichergestellt ist, dass nur ein zur Aufnahmehülse gehörender Stift 4 in der Aufnahmehülse desinfiziert werden kann. Es können auch spezielle, auf den zugehörigen Stift 4 abgestimmte Sensoren sowohl im Bereich der Freimachung 9 für den Minenkopf 8 als auch im Bereich der Aufnahmefläche 11 vorgesehen sein, um die ausschließliche Verwendung eines auf die Aufnahmehülse abgestimmten Stiftes 4 sicher zu stellen. Anstelle eines Sensors kann auch ein einfacher mechanischer Schalter verwendet werden. Ebenso können elektrische Kontaktflächen im Bereich der Freimachung 9 vorgesehen sein, die mit einer elektrisch leitenden Kugelschreibermine zusammenwirken.

Der Aufnahmeblock 10 bildet den oberen Abschluss eines Auswerfers, der aus dem Aufnahmeblock 10 und dem unterhalb des Aufnahmeblocks 10 angeordneten Teleskopzylinders 12 besteht. Der Teleskopzylinder 12 kann hydraulisch, elektrohydraulisch, pneumatisch oder elektrisch betrieben werden. Es sind sämtliche gängigen Arten von Teleskopzylindern prinzipiell für die Verwendung an der erfindungsmäßigen Aufnahmehülse geeignet.

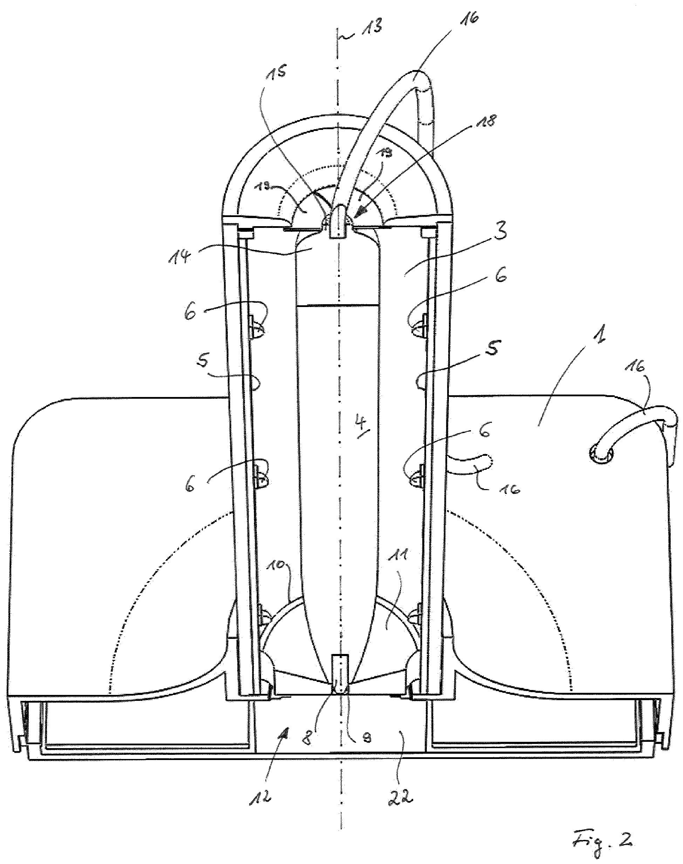

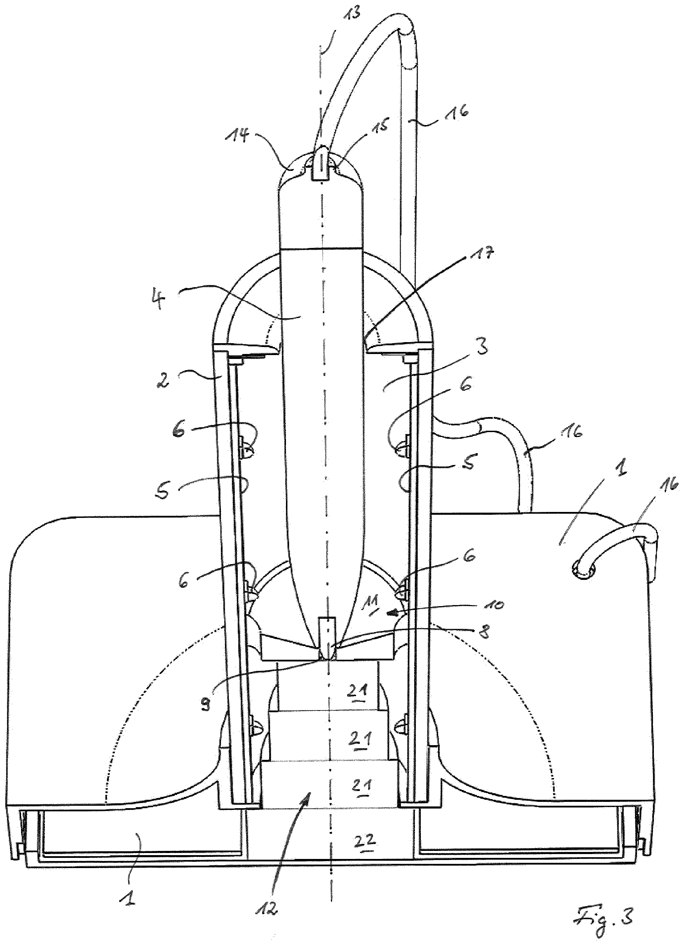

Aus dem in Richtung der Mittellängsachse 13 (Fig. 2) des Stiftes 4 der Stiftspitze 7 abgewandten Schaftende 14 des Stiftes 4 steht senkrecht ein Zentriervorsprung 15 hinaus. Der Zentriervorsprung 15 weist seinerseits eine Durchgangsöffnung auf, durch welche eine Halteschnur 16 hindurchgefädelt ist. Dieser Zentriervorsprung 15 ist so dimensioniert, dass die Hand des Bedieners mit der nachfolgend beschriebenen Halteschnur 16 beim Schreiben möglichst nicht in Berührung oder anderweitig in Kontakt kommt. Während das eine Ende der Halteschnur 16 am Stift 4 im Bereich des Zentriervorsprungs 15 adaptiert ist, ist das andere Ende der Halteschnur 16 am Standfuß 1 fixiert. Dies ist rechts oben am Standfuß in Fig. 2 bzw. Fig. 3 erkennbar.

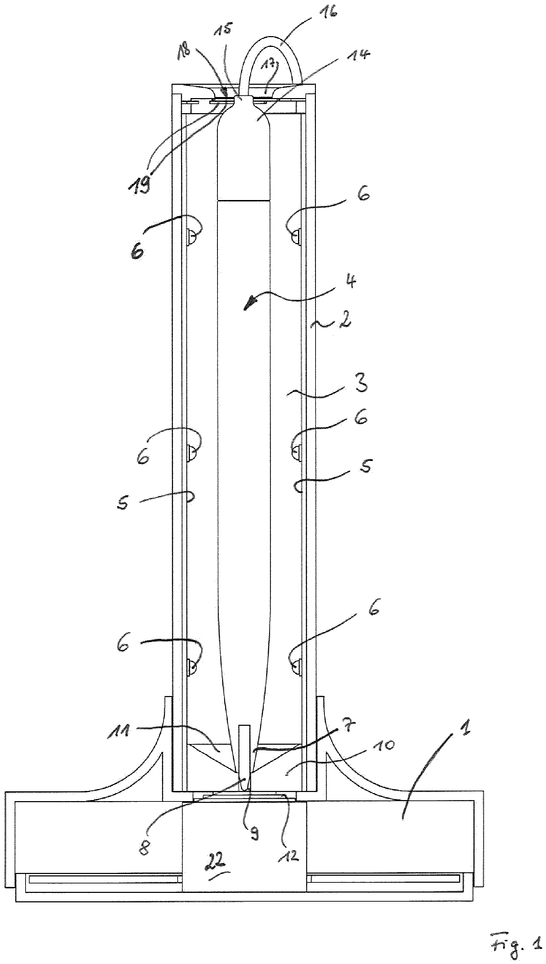

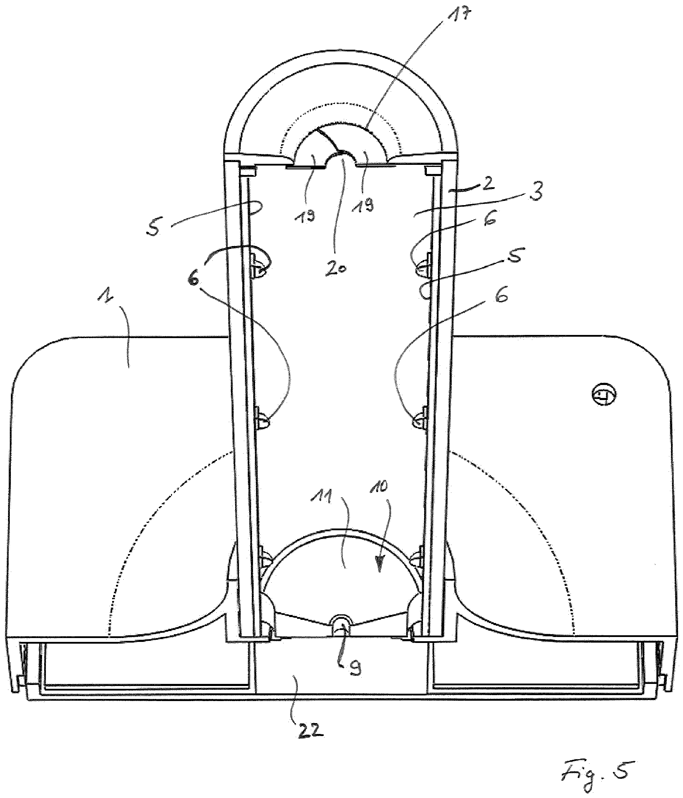

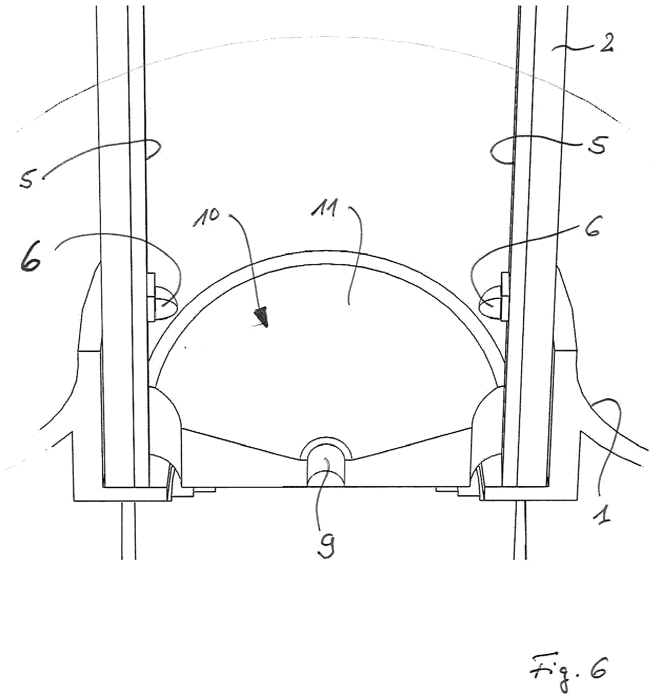

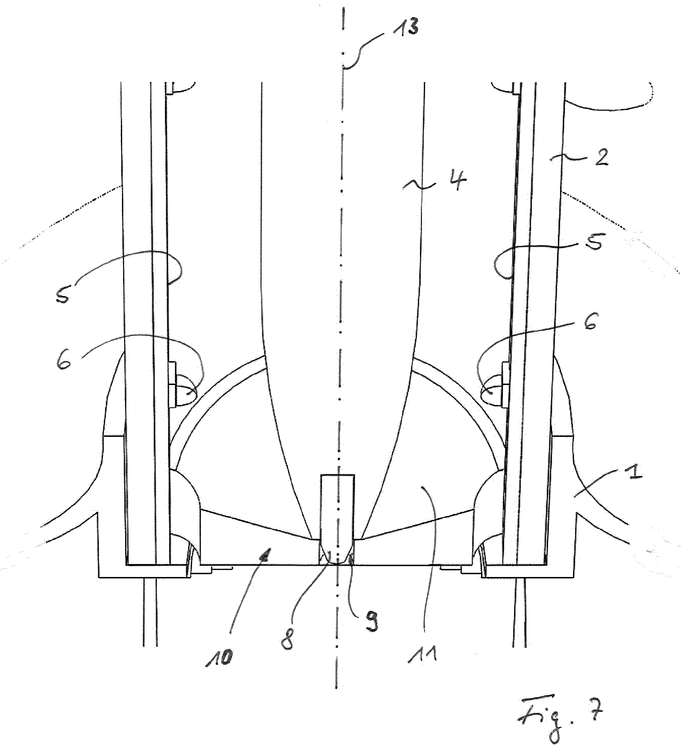

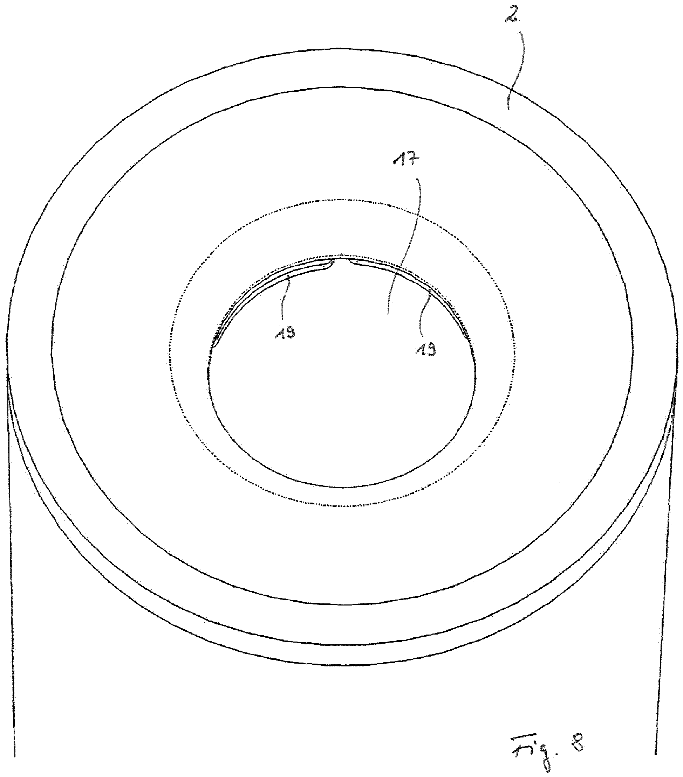

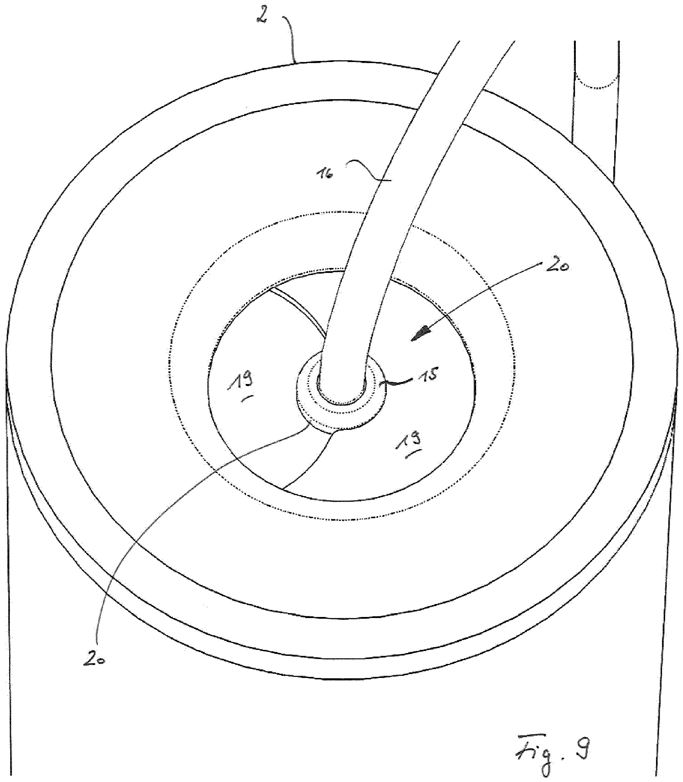

Der die Einführöffnung 17 am freien Aufnahmekammerende verschließende Verschluss 18 besteht im Ausführungsbeispiel der Fig. 1 - Fig. 9 aus zwei im verschlossenen Zustand überlappenden Lamellen 10. Die überlappenden Lamellen 10 lassen im verschlossenen Zustand eine Zentrieröffnung 20 im Verschluss 18 frei. Der Stift 4 durchsetzt bei verschlossenem Verschluss 18 mit dem Zentriervorsprung 15 den Verschluss 18 in dieser Zentrieröffnung 20 derart, dass die Lamellen 19 den Zentriervorsprung 15 allseitig umfassen und flankieren und auf diese Weise den Stift 4 in der Aufnahmekammer 3 zentrieren. Auf diese Weise ist die Mittellängsachse 13 sowohl die Mittellängsachse 13 des Stiftes 4 als auch die Mittellängsachse 13 der Aufnahmekammer 3. Beide Mittelängsachsen 13 sind also deckungsgleich.

Aus der Darstellung der Fig. 1 und der Fig. 2 ist erkennbar, dass der Stift sowohl mittels seines Zentriervorsprungs 15 im Verschluss 18 als auch mittels der Stiftspitze 7, nämlich mittels des Minenkopfes 8 in der Freimachung 9 zentriert ist. Auf diese Weise erreicht die von den Strahlungsquellen 6 emittierte UVC-Strahlung sämtliche Bereiche der Mantelfläche bzw. Außenfläche des Stifts 4. Der Stift 4 wird also vollständig von der UVC-Strahlung beaufschlagt. Hierbei unterstützt sowohl die reflektierende Aufnahmefläche 11 als auch die reflektierende Oberfläche der Innenwand 5 als auch die der reflektierenden Innenfläche der Aufnahmekammer 3 zugewandten Seiten der Lamellen 19 die Lenkung der Strahlung auf den Außenmantel des Stiftes 4.

Fig. 1 und Fig. 2 zeigen den Stift in seiner Desinfektionsstellung in der Aufnahmekammer 3. Nach dem Desinfizieren mit Hilfe der UVC-Strahlung der Strahlungsquellen 6 wird der Auswerfer aktiv. Hierfür wird der Teleskopzylinder 12 ausgefahren. In Fig. 3 ist erkennbar, wie die im Ausführungsbeispiel drei Teleskopelemente 21 aus dem Teleskopzylinderfuß 22 in Richtung der Mittellängsachse 13 nach oben ausfahren und so den Stift 4 durch die Einführöffnung 17 nach oben aus der Aufnahmekammer 3 herausdrücken bzw. heraus fahren. Sowohl das Schaftende 14 des Stifts 4 als auch ein Teil seines Schafts stehen aus der Aufnahmehülse hervor und können einfach vom Bediener mit der Hand gegriffen werden. Der vollständig desinfizierte Stift kann also auf diese Weise leicht vom Bediener mit der Hand aus der Aufnahmehülse herausgezogen werden.

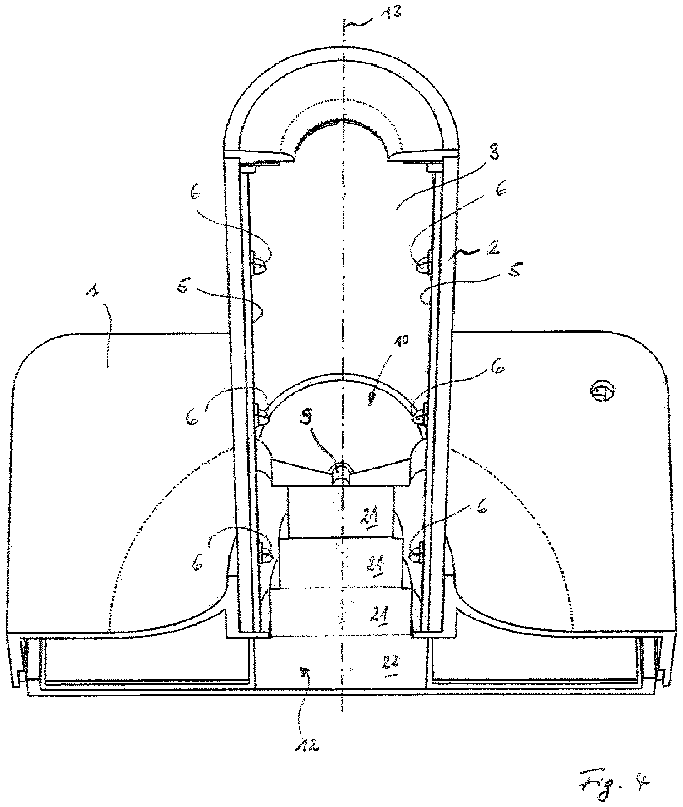

In Fig. 4 sind der Standfuß und der Hülsenkörper der erfindungsmäßigen Aufnahmehülse noch einmal abgebildet. Der Stift 4 ist herausgezogen und vollständig entfernt, weshalb auch die Halteschnur 16 in Fig. 4 nicht mehr erkennbar ist.

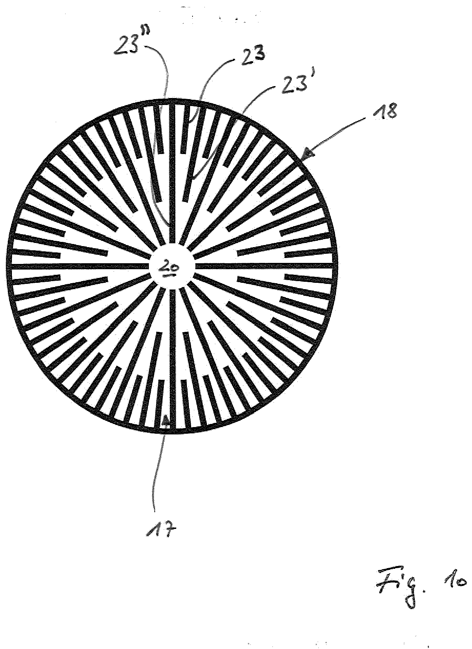

In Fig. 10 ist eine alternative Ausführungsform des Verschlusses 18 gezeigt. Erkennbar ist wiederum die Zentrieröffnung 20 im Verschluss 18. Der in Fig. 10 dargestellte Verschluss 18 besteht aus drei Bürstenringen mit verschieden langen Borsten 23, 23' und 23". Die Borsten 23, 23' und 23" stehen vom Öffnungsrand der Einführöffnung 17 radial in die Einführöffnung 17 hinein. Die Borsten 23" sind die längsten Borsten und reichen mit ihren freien Enden bis zur Zentrieröffnung 20. Die freien Enden der langen Borsten 23" flankieren die Zentrieröffnung 20 und bilden somit den Öffnungsrand der Zentrieröffnung 20. Die Borsten 23' mittlerer Länge enden mit deutlichem radial Abstand vor der Zentrieröffnung 20 und die kurzen Borsten 23 befinden sich lediglich im Bereich des Randes der Einführöffnung 17. Die verschieden langen Borsten 23, 23' und 23" bewirken dabei, dass die Einführöffnung 17 vollständig verschlossen ist.

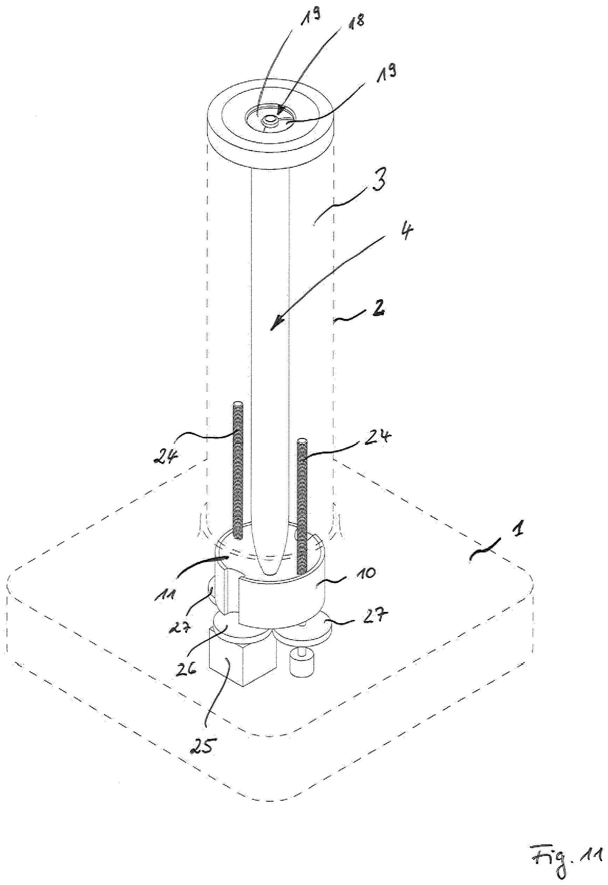

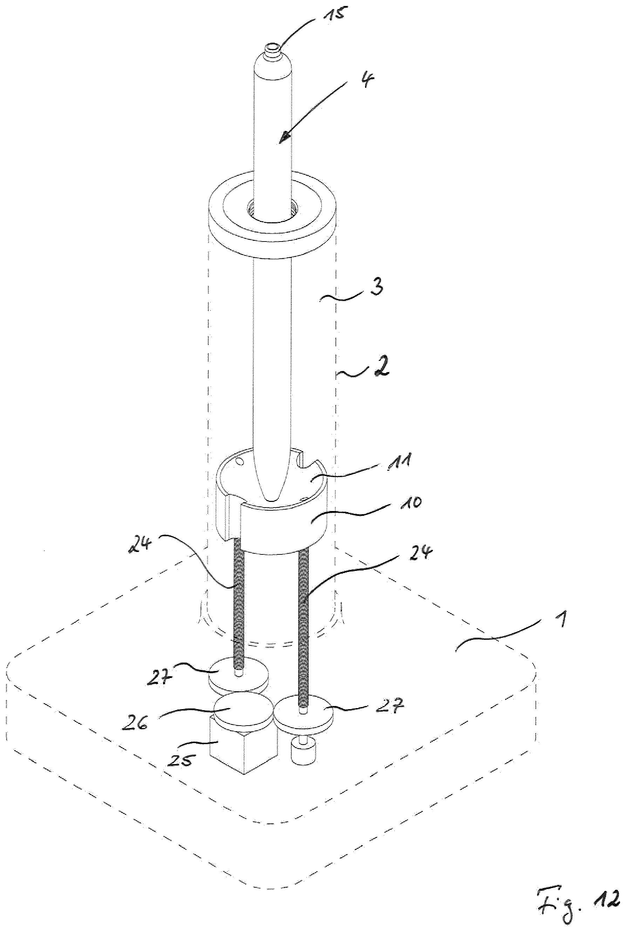

Fig. 11 und Fig. 12 zeigen eine weitere Ausführungsform des Auswerfers. Auch dieser Auswerfer besteht aus dem vorbeschriebenen Aufnahmeblock 10 mit der Aufnahmefläche 11. Anstelle des vorbeschriebenen Teleskopzylinders 12 treiben zwei einander gegenüberliegende Gewindestangen 24 den Aufnahmeblock 10 zur Verschiebung entlang der Mittellängsachse 13 an. Die Gewindestangen 24 durchsetzen hierfür den Aufnahmeblock 10 und greifen mit ihren Aussengewinden in entsprechende Innengewinde im Aufnahmeblock 10 ein. Die Gewindestangen 24 sind von einem Elektromotor 25 drehbar angetrieben. Der Elektromotor 25 weist hierfür ein drehendes Antriebszahnrad 26 auf, welches von jeweils einem an jeder Geweindestange 24 angeordneten Abtriebszahnrad 27 zu einem Getriebe ergänzt wird.

Fig. 11 zeigt den Stift 4 in seiner Desinfektionsstellung in der Aufnahmekammer 3. Nach dem Desinfizieren mittels der UVC-Strahlung der Strahlungsquellen 6 wird der Aufnahmeblock 10 von den vom Elektromotor 25 angetriebenen und als Antriebsspindeln wirksamen Gewindestangen 24 in Richtung der Mittellängsachse 13 nach oben in die in Fig. 12 dargestellte Entnahmestellung verfahren. Der Stift 4 kann nach Erreichen der Entnahmestellung in Fig. 12 aus der Aufnahmehülse entnommen werden.

### Bezugszeichenliste

- 1: Standfuß
- 2: Hülsenkörper
- 3: Aufnahmekammer
- 4: Stift
- 5: Innenwand
- 6: Strahlungsquelle
- 7: Stiftspitze
- 8: Minenkopf
- 9: Freimachung
- 10: Aufnahmeblock
- 11: Aufnahmefläche
- 12: Teleskopzylinder
- 13: Mittellängsachse
- 14: Schaftende
- 15: Zentriervorsprung
- 16: Halteschnur
- 17: Einführöffnung
- 18: Verschluss
- 19: Lamelle
- 20: Zentrieröffnung
- 21: Teleskopelement
- 22: Teleskopzylinderfuß
- 23, 23', 23": Borste
- 24: Gewindestange
- 25: Elektromotor
- 26: Antriebszahnrad
- 27: Abtriebszahnrad

## Patentansprüche

1. Aufnahmehülse für einen Stift (4) mit einer den Stift (4) über seine gesamte Länge aufnehmenden Aufnahmekammer (3) mit einer reflektierenden Innenwand (5) und mit einer oder mehreren Strahlungsquellen (6) an der Innenwand (5)
**gekennzeichnet**
• **durch** eine verschließbare Einführöffnung (17) am freien Aufnahmekammerende (3),
• **durch** einen Auswerfer an dem der Einführöffnung (17) abgewandten festen Ende der Aufnahmekammer (3) und
• **durch** mindestens ein Zentrierelement zur Zentrierung des Stiftes (4) entlang der Mittellängsachse (13) der Aufnahmekammer (3).

2. Aufnahmehülse nach Anspruch 1
**gekennzeichnet durch**
einen aus zwei oder mehr einander teilweise überlappenden Lamellen (19) gebildeten Verschluss (18) für die Einführöffnung (17).

3. Aufnahmehülse nach Anspruch 2
**gekennzeichnet durch**
eine der Aufnahmekammer (3) zugewandte reflektierende Seite der Lamellen (19).

4. Aufnahmehülse nach Anspruch 1
**gekennzeichnet durch**
mindestens einen Bürstenring mit Borsten (23, 23', 23") als Verschluss (18) für die Einführöffnung (17).

5. Aufnahmehülse nach Anspruch 1
**gekennzeichnet durch**
einen aus flexiblen Lippen gebildeten Verschluss (18) für die Einführöffnung (17).

6. Aufnahmehülse nach einem der Ansprüche 1 bis 5
**gekennzeichnet durch**
eine zentrale Zentrieröffnung (20) im Verschluss (18) als Aufnahme für einen am Stift (4) ausgebildeten Zentriervorsprung (15).

7. Aufnahmehülse nach Anspruch 6
**gekennzeichnet durch**
eine am Zentriervorsprung (15) adaptierte Halteschnur (16).

8. Aufnahmehülse nach einem der Ansprüche 1 bis 7
**gekennzeichnet durch**
einen Auswerfer mit einem Aufnahmeblock (10) und einer trichterförmigen Aufnahmefläche (11) für die Stiftspitze (7) am Aufnahmeblock (10), wobei die trichterförmige Aufnahmefläche (11) vorzugsweise eine zentrale Freimachung (9) für den Minenkopf (8) einer Stiftspitze (7) aufweist.

9. Aufnahmehülse nach einem der Ansprüche 1 bis 8
**gekennzeichnet durch**
einen Hubzylinder bzw. Teleskopzylinder (12) als Auswerfer.

10. Aufnahmehülse nach einem der Ansprüche 1 bis 8
**gekennzeichnet durch**
einen von Gewindestangen (24) angetriebenen, längsverschieblichen Aufnahmeblock (10) als Auswerfer.

11. Aufnahmehülse nach einem der Ansprüche 8 bis10
**dadurch gekennzeichnet**
**dass** der Auswerfer elektromotorisch angetrieben ist.
